# EUROPÄISCHE PATENTSCHRIFT

(11) **EP 3 787 575 B1**
(45) Veröffentlichungstag und Bekanntmachung des Hinweises auf die Patenterteilung: **17.01.2024**
(21) Anmeldenummer: 19722842.2
(22) Anmeldetag: 03.05.2019
(51) Int. Cl.: A61F 6/04

(54) **PRÄSERVATIV FÜR MÄNNER, FORMKÖRPER ZUM HERSTELLEN EINES PRÄSERVATIVS FÜR MÄNNER UND HERSTELLUNG EINES PRÄSERVATIVS FÜR MÄNNER**
CONTRACEPTIVE FOR MEN, MOULD BODY FOR PRODUCING A CONTRACEPTIVE FOR MEN, AND PRODUCTION OF A CONTRACEPTIVE FOR MEN
PRÉSERVATIF POUR HOMME, CORPS MOULÉ PERMETTANT DE FABRIQUER UN PRÉSERVATIF POUR HOMME ET FABRICATION D'UN PRÉSERVATIF POUR HOMME

(30) Priorität: 03.05.2018 DE 102018206856
(43) Veröffentlichungstag der Anmeldung: 10.03.2021
(73) Patentinhaber: Sanavita Pharmaceuticals GmbH, 20097 Hamburg (DE)
(72) Erfinder: ANTUNES COXILHA, Denis, 21035 Hamburg (DE)
(74) Vertreter: Hoffmann Eitle
(86) Internationale Anmeldenummer: PCT/EP2019/061368
(87) Internationale Veröffentlichungsnummer: WO 2019/211433

(56) Entgegenhaltungen:
- DE-A1- 4 231 797
- US-A- 4 852 586
- US-A- 5 836 308
- US-A1- 2007 181 134
- US-A1- 2015 157 494
- US-B1- 6 298 853

## Beschreibung

### Technisches Gebiet

Die Erfindung betrifft ein Präservativ für Männer, einen Formkörper zum Herstellen eines Präservativs sowie ein Verfahren zur Herstellung eines Präservativs, vorzugsweise aus Latex.

### Stand der Technik

Präservative (auch genannt Kondome) für Männer dienen zur Empfängnisverhütung und zum Schutz gegen sexuell übertragbare Krankheiten. Solche Präservative werden im aufgerollten Zustand verpackt und müssen für die Anwendung ausgerollt werden. Beim Abrollen und Anlegen auf den Penis ist es wichtig, dass das Präservativ mit der Außenseite nach außen zeigt und nicht fälschlicherweise die Innenseite nach außen zeigt, da dann der Rand, der beim Aufrollen ausgebildet wurde, praktisch nicht abgerollt werden kann. Es ist also wesentlich, dass die Außenseite des Präservativs nach der Entnahme aus der Verpackung leicht identifiziert werden kann. Unter der Außenseite ist die Seite des Präservativs zu verstehen, die dafür vorgesehen ist, bei Verwendung nicht in Kontakt mit dem Penis zu sein bzw. weg vom Penis zu zeigen. Die Innenseite des Präservativs ist dafür vorgesehen, bei Verwendung am Penis anzuliegen.

Um in diesem Zusammenhang Verbesserungen zu schaffen, schlägt die CN 107374809 A vor, eine farbige Schicht in das Präservativ einzufügen, sodass optisch zwischen der Außenseite und der Innenseite unterschieden werden kann. Derartige Präservative können jedoch nicht ausreichend Verbesserung schaffen, sodass höhere Anforderungen an die Handhabbarkeit eines Präservativs gestellt werden.

DE 42 31 797 A1 offenbart ein Kondom mit einem Abschnitt am geschlossenen Kondomende mit Erhebungen in einer regelmäßigen geometrischen Struktur als Abrollmarkierung.

US 5,836,308 A offenbart ein Kondom mit einer federartigen Struktur, die relativ zum Kondom beweglich ist.

US 2007/0181134 A1 offenbart ein Verfahren zum Herstellen eines Kondoms und ein Kondom gemäß dem Oberbegriff von Anspruch 1.

US 2015/0157494 A1 offenbart ein Kondom, das einen elliptischen Rand aufweist, der einen Teil der Vulva der Frau überlappt.

US 4,852,586 offenbart ein Kondom mit mehreren entgegengesetzten Paaren von Vorsprüngen, wobei eine Membran als Stützpunkt für die Übertragung der Bewegung eines der Vorsprünge eines Paares auf den anderen Vorsprung des Paares wirkt.

US 6,298,853 B1 offenbart ein verkürztes Kondom, das auf der Eichel des Penis positioniert wird.

### Darstellung der Erfindung

Es ist Aufgabe der vorliegenden Erfindung, bei einfacher Konstruktion die Handhabbarkeit eines Präservativs bzw. Kondoms zu verbessern.

Diese Aufgabe wird erfindungsgemäß durch das Präservativ des Anspruchs 1 gelöst. Demzufolge ist insbesondere ein Präservativ für Männer vorgesehen, das sich in zumindest einem Zustand im Wesentlichen entlang einer Längsachse erstreckt und einen ersten Abschnitt und einen zweiten Abschnitt aufweist, wobei der zweite Abschnitt insbesondere zur Aufnahme von Sperma dient. Der zweite Abschnitt ist an einem Ende des Präservativs entlang der Längsachse betrachtet angeordnet und weist zumindest abschnittsweise eine Oberflächenstruktur auf der Außenseite des Präservativs auf.

Die Oberflächenstruktur ist derart eingerichtet, dass die haptische Wahrnehmbarkeit durch Finger erhöht ist. Die Oberflächenstruktur ist derart ausgelegt, dass sie mit den Fingern gegriffen werden kann. Anders ausgedrückt ist die Oberflächenstruktur mit den Fingern haptisch wahrnehmbar bzw. mit den Fingern tastbar. Die Oberflächenstruktur kann dabei die Griffigkeit erhöhen. Die Oberflächenstruktur kann eine Textur sein und/oder kann ein Muster aufweisen.

Es ist nicht ausgeschlossen, dass der erste Abschnitt frei von einer Oberflächenstruktur ist. Der erste Abschnitt kann aber frei von einer, insbesondere frei von der erfindungsgemäßen Oberflächenstruktur im zweiten Abschnitt sein. Die Oberflächenstruktur im zweiten Abschnitt ist vorzugsweise unterschiedlich zu einer gegebenenfalls im ersten Abschnitt vorliegenden Oberflächenstruktur.

Die Oberflächenstruktur im zweiten Abschnitt erstreckt sich vorzugsweise um weniger aus ein Drittel, weiter vorzugsweise um weniger als ein Viertel, Fünftel oder Sechstel der Gesamtlänge des Präservativs in die Längsrichtung. Anders ausgedrückt sind zumindest zwei Drittel, drei Viertel, vier Fünftel oder fünf Sechstel der Gesamtlänge des Präservativs in Längsrichtung frei von der erfindungsgemäßen Oberflächenstruktur.

Bei dem zweiten Abschnitt handelt es sich um ein Reservoir zur Aufnahme von Sperma. Der zweite Abschnitt dient also zur Aufnahme von Sperma, d.h. als Reservoir. Der zweite Abschnitt ist vorzugsweise distal zum ersten Abschnitt angeordnet. Bei dem Ende des Präservativs handelt es sich um das geschlossene Ende des Präservativs, an dem ein Reservoir zum Aufnehmen von Sperma ausgebildet sein kann. Das Ende des Präservativs ist geschlossen. Der erste und der zweite Abschnitt können sich entlang der Längsachse erstrecken. Der erste Abschnitt ist vorzugsweise an dem offenen Ende angeordnet und der zweite Abschnitt an dem offenen Ende des Präservativs entlang der Längsrichtung. Der erste Abschnitt ist im Wesentlichen zylinderförmig ausgebildet. Auch der zweite Abschnitt kann zylinderförmig ausgebildet sein. Insbesondere kann der erste und/oder zweite Abschnitt in zumindest einem Zustand zylinderförmig ausgebildet sein.

Des Weiteren ist gemäß Anspruch 5 ein erfindungsgemäßer Formkörper zum Herstellen eines Präservativs für Männer vorgesehen. Der Formkörper erstreckt sich im Wesentlichen entlang einer Formlängsachse und weist einen ersten Formabschnitt und einen zweiten Formabschnitt auf. Der erste Formabschnitt dient insbesondere zum Ausbilden des ersten Abschnitts des Präservativs und der zweite Formabschnitt dient insbesondere zum Ausbilden des zweiten Abschnitts des Präservativs. Insbesondere sind die Längsachse des Präservativs und die Formlängsachse des Formkörpers identisch. Der zweite Formabschnitt ist an einem Ende des Formkörpers entlang der Formlängsachse betrachtet angeordnet und weist auf der Außenseite zumindest abschnittsweise zumindest eine Oberflächenformstruktur auf.

Die Oberflächenformstruktur kann zur Ausbildung der Oberflächenstruktur des erfindungsgemäßen Präservativs dienen. Der Formkörper ist insbesondere ein Negativ für das Präservativ. Folglich gilt das für das Präservativ bzw. den Formkörper Gesagte entsprechend für den Formkörper bzw. für das Präservativ.

Des Weiteren ist erfindungsgemäß ein Verfahren zur Herstellung eines Präservativs gemäß Anspruch 9 vorgesehen. Demzufolge wird ein sich im Wesentlichen entlang einer Längsachse erstreckender Formkörper bereitgestellt und dann der Formkörper zumindest abschnittsweise in flüssiges Material zum Ausbilden eines Materialfilms zumindest abschnittsweise um den Formkörper herum eingetaucht. Gleichzeitig oder danach wird eine Oberflächenstruktur im Materialfilm zumindest an dem am tiefsten eingetauchten Bereich des Formkörpers ausgebildet.

Die Oberflächenstruktur des Präservativs, insbesondere im zweiten Abschnitt des Präservativs, wird vorzugsweise dadurch ausgebildet, dass sich das flüssige Material um eine entsprechende Oberflächenstruktur des Formkörpers legt. Der tiefste eingetauchte Bereich des Formkörpers kann also der zweite Formabschnitt des Formkörpers sein, der an einem Ende des Formkörpers entlang der Formlängsachse betrachtet angeordnet ist und auf der Außenseite zumindest abschnittsweise zumindest eine Oberflächenformstruktur aufweist. Vorzugsweise ist ein weniger tief eingetauchter Bereich des Formkörpers frei von der Oberflächenstruktur.

Eine Oberflächenstruktur des Präservativs muss nicht ausschließlich auf der Außenseite angeordnet sein, sondern kann auch auf der Innenseite ausgebildet sein. Die Oberflächenstruktur auf der Innenseite kann der erfindungsgemäßen Oberflächenstruktur auf der Außenseite des Präservativs entsprechen. Beispielsweise ist es denkbar, dass die Oberflächenstruktur oder eine der im Wesentlichen auf der Außenseite vorliegenden Oberflächenstruktur entsprechende Oberflächenstruktur auf der Innenseite vorliegt. Insbesondere kann die Oberflächenstruktur auf der Innenseite einer Inversion der Oberflächenstruktur auf der Außenseite entsprechen.

Das Präservativ kann aus Latex, insbesondere Naturkautschuklatex, oder Polyurethan bestehen. Die Oberflächenstruktur ist insbesondere aus dem gleichen Material wie das Präservativ als solches ausgebildet, das heißt insbesondere aus Latex bei einem Latex-Präservativ bzw. Polyurethan bei einem Polyurethan-Präservativ.

Die Wandstärke des Präservativs beträgt etwa weniger als 0,1 mm, bevorzugt zwischen 0,08 mm und 0,04 mm. Vorzugsweise ist die Wandstärke auch im Bereich der Oberflächenstruktur im Wesentlichen gleich. Es ist aber denkbar, dass die Wandstärke zumindest teilweise in diesem Bereich etwas höher ausfällt.

Insbesondere handelt es sich bei dem erfindungsgemäßen Präservativ bzw. Kondom um ein Medizinprodukt der Klasse IIb gemäß der Richtlinie 93/42/EWG, und nicht um ein sogenanntes Scherzkondom. Es ist also vorzugsweise als medizinisches Präservativ zu bezeichnen. Das erfindungsgemäße Präservativ erfüllt die Anforderungen gemäß dem ISO-Standard 4074 (2015).

Der Erfindung liegt der Gedanke zu Grunde, das Abrollen eines Präservativs dadurch zu vereinfachen, dass die Außenseite des Präservativs durch die Oberflächenstruktur ertastbar und somit leicht identifizierbar wird. Gleichzeitig dient die Oberflächenstruktur dazu, dass das Präservativ besser mit den Finger gegriffen und gehalten werden kann, wenn das Präservativ abgerollt wird. Dies trägt dazu bei, die Handhabbarkeit des Präservativs zu erhöhen.

Die Oberflächenstruktur ist insbesondere auch vor dem Hintergrund vorteilhaft, dass Präservative üblicherweise beschichtet sind, beispielsweise mit Silikongel. Somit ist die Handhabung des Präservativs oft dadurch erschwert, dass das Präservativ als solches von Gleitmittel umgeben ist. Die erfindungsgemäße Oberflächenstruktur kann auch in diesem Fall die Griffigkeit verbessern.

Durch die verbesserte Handhabbarkeit ergeben sich auch Vorteile hinsichtlich der Sicherheit, da vermieden werde kann, dass beispielsweise das Präservativ vor dem Anziehen mit den Fingern umgedreht wird oder falschherum angelegt wird. Denn dadurch könnte das Präservativ beschädigt werden und der gewünschte Schutz nicht ausreichend gewährleistet sein. Beispielsweise kann auch vermieden werden, dass das Präservativ mit der Außenseite nach innen zeigend falsch herum angelegt wird und dabei die eigentliche Außenseite mit dem Penis und insbesondere mit Sperma in Kontakt kommt. Denn wenn das Präservativ im Anschluss umgedreht und richtig herum angezogen wird, zeigt die Außenseite, die mit dem Sexualpartner in Kontakt kommt (richtigerweise) nach außen, kann aber mit Sperma und/oder Körperflüssigkeit des Mannes behaftet sein. Dadurch würde die Schutzwirkung des Präservativs was Empfängnisverhütung und Schutz gegen sexuell übertragbare Krankheiten nicht mehr gegeben sein.

Die Erfindung ist insbesondere auch dann vorteilhaft, wenn kein oder wenig Licht vorhanden ist, was für eine optische Identifizierung der Außenseite ja unabdingbar ist.

Auch stimulierende Effekte insbesondere bei der Frau können sich durch die erfindungsgemäße Oberflächenstruktur ergeben.

Der zweite Abschnitt stellt einen Abschnitt dar, der zur Aufnahme von Sperma dient. Dieser Bereich ist beim Anlegen des Präservativs frei von Luft zu halten, was dadurch erreicht wird, dass der zweite Abschnitt, oft auch Reservoir genannt, beim Anziehen des Präservativs mit den Fingern zusammengedrückt wird. Auch unter diesem Aspekt ist es vorteilhaft, dass dieser Abschnitt die erfindungsgemäße Oberflächenstruktur aufweist.

Vorteilhafte Weiterbildungen der Erfindung sind in den abhängigen Ansprüchen angegeben.

Vorzugsweise ist die Oberflächenstruktur in einem Bereich zweiten Abschnitts angeordnet, wo die Oberfläche des zweiten Abschnitts parallel oder im Wesentlichen parallel zur Längsachse verläuft. Es ist also auch angedacht, dass der Bereich der Oberfläche, der senkrecht oder im Wesentlichen senkrecht zur Längsachse verläuft, frei von der Oberflächenstruktur ist. Anders ausgedrückt kann ein Bereich, der leicht winklig zur Längsachse verläuft, vorzugsweise in einem Bereich von weniger als 30° zur Längsachse, die Oberflächenstruktur aufweisen. Dadurch kann erreicht werden, dass insbesondere ein Bereich die Oberflächenstruktur aufweist, in dem die Finger beim Anlegen des Präservativs ansetzen. Ebenso kann die Oberflächenformstruktur des Formkörpers in einem Bereich des zweiten Formabschnitts angeordnet sein, der sich parallel oder im Wesentlichen parallel zur Formlängsachse erstreckt. Der zweite Formabschnitt kann also an seiner Spitze frei von der Oberflächenformstruktur sein.

Die Erfindung sieht vor, dass ein Übergang vom ersten Abschnitt zum zweiten Abschnitt vorliegt und dieser Übergang eine Verjüngung aufweist, sodass sich das Präservativ vom ersten Abschnitt zum zweiten Abschnitt hin verjüngt. Entsprechend weist der Formkörper einen Formübergang zwischen dem ersten und dem zweiten Formabschnitt auf, wobei der Formübergang eine Formverjüngung vom ersten Formabschnitt zum zweiten Formabschnitt aufweist, sodass sich der Formkörper vom ersten Formabschnitt zum zweiten Formabschnitt verjüngt.

Der zweite Abschnitt entspricht in diesem Fall insbesondere dem sogenannten Reservoir, das zur Aufnahme von Sperma dient. Üblicherweise ist dieser zweite Bereich bzw. Abschnitt schmäler, d.h. der Durchmesser ist im Vergleich zum Durchmesser des ersten Abschnitts zumindest um die Hälfte, bevorzugt um mindestens zwei Drittel, weiter bevorzugt um mindestens drei Viertel, reduziert. Typischerweise beträgt der Durchmesser des verjüngten zweiten Abschnitts weniger als 20 mm, bevorzugt etwa 15 mm, und der des ersten Abschnitts mindestens 45 mm, bevorzugt zwischen 45 und 58 mm, weiter vorzugsweise zwischen 49 und 56 mm im ausgerollten Zustand. Die Oberflächenstruktur weist auf der Außenseite mehrere Vorsprünge auf. Gleichermaßen weist die Oberflächenformstruktur des Formkörpers mehrere Formvorsprünge auf der Außenseite des Formkörpers auf. Das kann dazu führen, dass sich ein Vorsprung bzw. Vorsprünge auf der Außenseite als Vertiefung(en) auf der Innenseite des Präservativs widerspiegeln. Durch die Ausbildung eines Vorsprungs auf der Außenseite des Präservativs kann die haptische Wahrnehmung durch Finger, insbesondere durch Streichen der Finger, besonders gut erreicht werden. Dadurch ergibt sich also eine besonders vorteilhafte Handhabbarkeit des Präservativs.

Die Oberflächenstruktur weist mehrere Noppen und vorzugsweise mehrere Rippen auf. Anders ausgedrückt können also der Vorsprung bzw. die Vorsprünge als Noppen und vorzugsweise Rippen ausgebildet sein. Dies ist haptisch und fertigungstechnisch vorteilhaft. Vorzugsweise weist die Oberflächenstruktur zumindest drei, fünf, zehn, 15 oder 20 Noppen und vorzugsweise Rippen auf. Weiter sind diese in einem Muster in der Oberflächenstruktur angeordnet.

Die Anordnung der Noppen und vorzugsweise Rippen ist auf vielfältige Weise denkbar.

Die Erfindung sieht vor, dass die Vorsprünge als Noppen ausgebildet sind, wobei die Noppen derart gruppiert sind, dass die Gruppierung im Wesentlichen kreisförmig um die Längsachse verläuft. Vorzugsweise verlaufen mehrere Gruppierungen von Noppen parallel zueinander.

Die Formnoppen können auf entsprechende Weise derart kopiert sein, dass die Formgruppierung im Wesentlichen kreisförmig um die Formlängsachse und/oder winkelig zur Formlängsachse verläuft und vorzugsweise in mehreren Gruppierungen parallel zueinander verlaufen. Beispielsweise können zehn Formnoppen auf dem zweiten Formabschnitt angeordnet sein und beispielsweise gleichmäßig im zweiten Formabschnitt verteilt sein. Eine entsprechende Gestaltung der Noppen im Präservativ ergibt sich entsprechend.

Im Allgemeinen steht eine Noppe um weniger als 1 mm, bevorzugt zwischen 0.3 und 1.0 mm vor. Die Breite einer Noppe beträgt weniger als 2 mm bzw. die Breite einer Rippe beträgt vorzugsweise zwischen 0.5 mm und 2.0 mm. Entsprechende Abmessungen gelten für die Formnoppen bzw. Formrippen des Formkörpers.

Gemäß dem Verfahren zum Herstellen eines Präservativs wird der Formkörper, nachdem er in flüssiges Material eingetaucht worden ist und eine Oberflächenstruktur im am tiefsten eingetauchten Bereich des Formkörpers ausgebildet worden ist, der entstandene Materialfilm erwärmt, wodurch der Materialfilm getrocknet wird. Beispielsweise kann der Formkörper dazu mit Heißluft beaufschlagt werden.

Der Materialfilm bildet das Präservativ aus. Üblicherweise erfolgt das Eintauchen des Formkörpers in das flüssige Material drei Mal. Beim Eintauchen des Formkörpers wird der Formkörper von Material überzogen. Anders ausgedrückt legt sich Material um den Formkörper herum. Dieses Material bildet das Präservativ aus. Vorzugsweise ist das Material, in das der Formkörper eingetaucht wird, flüssiges Latex.

Nach dem Trocknen des Materialfilms kann der Film vulkanisiert werden. Nach einem Waschen- und Trockenvorgang wird der Materialfilm aufgerollt, sodass sich ein Rand ausbildet. Nun befindet sich nun das Präservativ in dem Zustand befindet, in dem es verpackt werden kann.

Insbesondere dient der erfindungsgemäße Formkörper zum Herstellen eines erfindungsgemäßen Präservativs, wobei der Formkörper dazu eingerichtet ist, in flüssiges Material, insbesondere flüssiges Latex, eingetaucht zu werden, sodass sich ein Latexfilm um den Formkörper herum ausbildet, der das Präservativ bildet.

Weitere Merkmale und Vorteile der Erfindung werden anhand der nachfolgenden ausführlichen Beschreibung noch näher ersichtlich werden.

### Kurze Beschreibung der Zeichnung

- Fig. 1a: zeigt ein nicht-erfindungsgemäßes Präservativ; Fig. 1b zeigt eine schematische Seitenansicht von Präservativen gemäß der Erfindung;
- Fig. 2: zeigt ein nicht-erfindungsgemäßes Präservativ im aufgerollten Zustand;
- Fig. 3a: zeigt ein nicht-erfindungsgemäßes Präservativ, bei dem die Außenseite im aufgerollten Zustand nach innen zeigt;
- Fig. 3b: zeigt ein nicht-erfindungsgemäßes Präservativ im aufgerollten Zustand, bei dem die Außenseite nach außen zeigt;
- Fig. 4a und 4b: zeigen einen nichterfindungsgemäßen Formkörper; und
- Fig. 5: zeigt eine nicht-erfindungsgemäße Ausführungsform eines erfindungsgemäßen Formkörpers.

Ausführliche Beschreibung bevorzugter Ausführungsformen Bevorzugte Ausführungsformen der vorliegenden Erfindung werden nachfolgend ausführlich unter Bezugnahme auf die begleitenden Zeichnungen beschrieben.

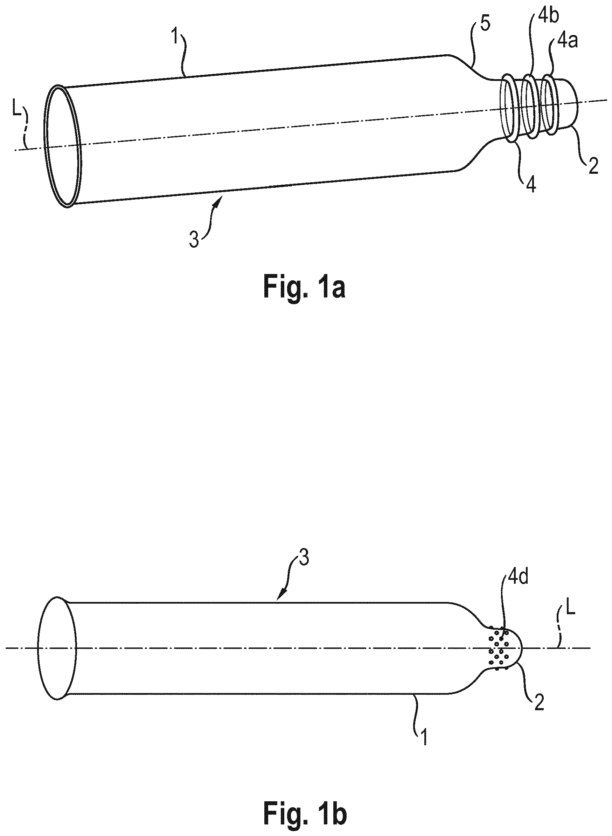

Figur 1a zeigt ein nicht-erfindungsgemäßes Präservativ 3 für Männer, das sich in zumindest einem Zustand, insbesondere im abgerollten und/oder angezogenen Zustand, im Wesentlichen entlang der Längsachse L erstreckt. Das Präservativ 3 weist einen ersten Abschnitt 1 und einen zweiten Abschnitt 2 auf. Der zweite Abschnitt 2 ist an einem Endabschnitt des Präservativs entlang der Längsachse L angeordnet, also an dem Bereich, an dem das Präservativ 3 geschlossen ist. An dem gegenüberliegenden Ende, an dem der erste Abschnitt 1 angeordnet ist, ist das Präservativ 3 offen. Das Präservativ 3 weist einen Übergang 5 auf, der eine Verjüngung vom ersten Abschnitt 1 zum zweiten Abschnitt 2 darstellt. Der zweite Abschnitt 2 ist also gegenüber dem ersten Abschnitt 1 verjüngt und hat einen kleineren Durchmesser.

In dem Bereich des zweiten Abschnitts 2, der im Wesentlichen parallel zur Längsachse L verläuft, ist die Oberflächenstruktur 4 ausgebildet. Die Oberflächenstruktur 4 ist auf der Außenseite des Präservativs angeordnet und derart eingerichtet, dass sie die haptische Wahrnehmbarkeit durch Finger, insbesondere bei Streicheln der Finger über die Oberfläche, erhöht. Damit kann die Oberflächenstruktur 4 mit den Fingern ertastet werden und die Griffigkeit erhöht werden, sodass der Anwender das Präservativ besser mit den Fingern greifen kann. Dadurch ergeben sich die vorne beschriebenen Vorteile.

In der hier gezeigten Ausführungsform ist das Präservativ 3 im ersten Abschnitt 1 frei von der Oberflächenstruktur 4 bzw. eine Oberflächenstruktur im ersten Abschnitt 1 ist nicht sichtbar. Im zweiten Abschnitt 2 ist die Oberflächenstruktur 4 durch als Rippen ausgebildete Vorsprünge 4a, 4b ausgebildet, die auf der Außenseite des Präservativs 3 vorliegen. In der Ausführungsform von Figur 1a liegen die Vorsprünge als drei Rippen vor, die konzentrisch um die Längsachse L angeordnet sind und den gleichen Abstand zueinander aufweisen, also parallel zueinander verlaufen.

Figur 1b zeigt ein erfindungsgemäßes Präservativ 3, das bis auf die Oberflächenstruktur 4 dem Präservativ entspricht, das in Figur 1a gezeigt ist. Anstelle der Vorsprünge 4a, 4b von Figur 1a sind in der Ausführungsform von Fig. 1b Noppen 4d ausgebildet. Diese sind in einem Muster angeordnet, in dem die Noppen zueinander versetzt angeordnet sind.

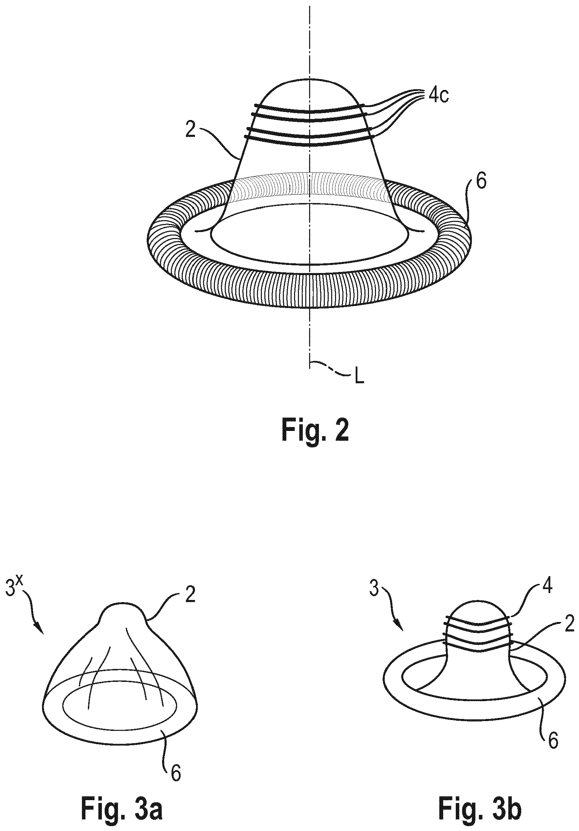

Figur 2 zeigt ein nicht-erfindungsgemäßes Präservativ im aufgerollten Zustand, in dem der Rand 6 ausgebildet ist. Auf dem zweiten Abschnitt 2 liegen vier Rippen 4c vor, die parallel zueinander verlaufen und konzentrisch um die Längsachse L angeordnet sind. Zwei der Rippen 4c weisen einen geringeren Abstand zueinander auf als zu einer weiteren benachbarten Rippe.

In Figur 3b ist im Wesentlichen das gleiche Präservativ 3 wie in Figur 2 gezeigt, wobei hier durch die Gegenüberstellung mit dem nicht-erfindungsgemäßen Präservativ 3^{x} gemäß Figur 3a Folgendes herausgestellt werden soll: Dadurch, dass durch die Oberflächenstruktur 4 des erfindungsgemäßen Präservativs 3 der Figur 3b die Außenseite des Präservativs schnell und zuverlässig identifiziert werden kann, kann das Präservativ 3 vor dem Anziehen mit seiner Außenseite nach außen positioniert werden, sodass sich der Rand 6 leicht abrollen lässt und damit das Präservativ in den abgerollten Zustand gebracht werden kann. In Figur 3a hingegen ist der Zustand gezeigt, in dem sich das Präservativ 3^{x} nicht abrollen lässt, nämlich der Zustand, in dem die Innenseite nach außen zeigt. Der Rand 6 wird sich hier nicht abrollen lassen, zumindest nicht ohne größere Schwierigkeiten. Dieser Zustand kann durch die Erfindung verhindert werden bzw. leicht identifiziert werden, sodass ein solcher Zustand schnell in den gewünschten "richtigen" Zustand gebracht werden kann. Um das Präservativ 3^{x} aus dem "falschen" Zustand von Figur 3a in den "richtigen" Zustand von Figur 3b zu bringen, ist es nötig, den zweiten Abschnitt 2 umzustülpen. Dazu gilt es aber zunächst herauszufinden, ob das Präservativ, im aufgerollten Zustand, richtig oder falsch positioniert ist, das heißt ob tatsächlich die Außenseite nach außen zeigt oder die Innenseite fälschlicherweise nach außen zeigt. Das Beispiel schafft hier durch die Oberflächenstruktur 4, wie sie in dem Präservativ 3 von Figur 3b gezeigt ist, den Vorteil, dass durch die haptische Wahrnehmbarkeit durch die Finger die Identifizierung vereinfacht wird. Gleichzeitig wird durch die Oberflächenstruktur ermöglicht, dass der zweite Abschnitt bessert gegriffen werden kann und ein Abrutschen der Finger von dem zweiten Abschnitt 2 beim Anlegen des Präservativs 3 verhindert wird.

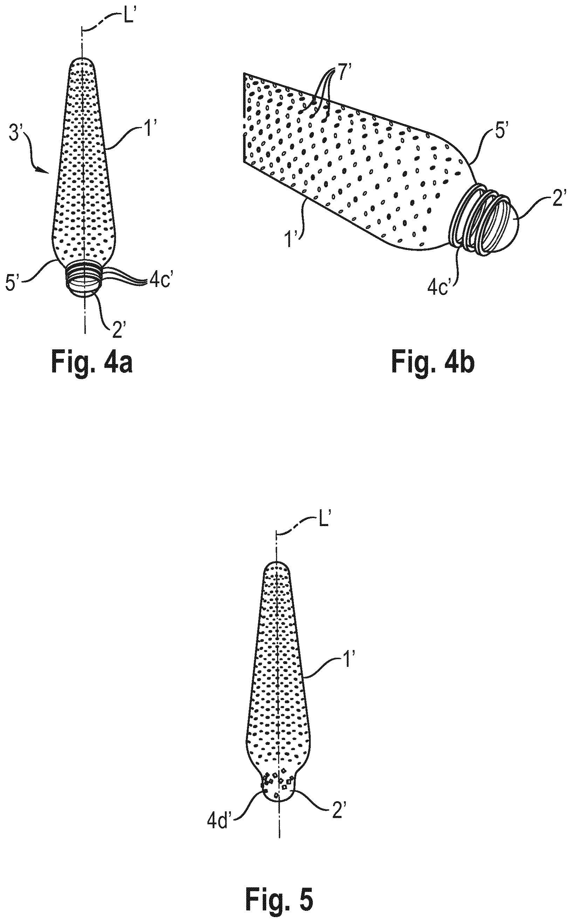

Die Figuren 4a und 4b zeigen einen nichterfindungsgemäßen Formkörper zum Herstellen eines nichterfindungsgemäßen Präservativs, wobei sich der Formkörper 3' im Wesentlichen entlang einer Formlängsachse L' erstreckt. Die Formlängsachse L` des Formkörpers 3' entspricht der Längsachse L des Präservativs 3.

Der Formkörper 3' weist einen ersten Formabschnitt 1` zum Ausbilden des ersten Abschnitts 1 des Präservativs 3 und einen zweiten Formabschnitt 2' zum Ausbilden des zweiten Abschnitts 2 des Präservativs 3 auf. Entsprechend dem in Figur 1 gezeigten Präservativ 3 weist der Formkörper 3' einen Formübergang 5' auf, der eine Verjüngung vom ersten Formabschnitt 1' zum zweiten Formabschnitt 2' darstellt.

Die gleichen Abmessungen wie für das Präservativ gelten hinsichtlich des Formkörpers, da das Präservativ 3 dem Film entspricht, der den Formkörper 3' umgibt.

Das Beispiel der Figuren 4a und 4b entspricht im Wesentlichen der Ausführungsform von Figur 1, da der Formkörper 3' drei Formrippen 4c' aufweist, die konzentrisch um die Längsachse L' verlaufen. Zusätzlich weist der in Figuren 4a und 4b gezeigte Formkörper 3' Seitenformnoppen 7' auf, die an dem ersten Formabschnitt 1` angeordnet sind. Diese Seitenformnoppen 7' führen zu einer entsprechenden Ausbildung von Seitennoppen 7 (nicht gezeigt) im Präservativ 3 im ersten Abschnitt 1. Wie vorne ausgeführt sind die Seitenformnoppen 7` nicht Teil der Oberflächenstruktur 4` des Formkörpers 3'.

Figur 5 zeigt eine weitere Ausführungsform eines nichterfindungsgemäßen Formkörpers 3', bei dem im zweiten Abschnitt 2' Formnoppen 4d' angeordnet sind. Die Formnoppen 4d' sind ungleichmäßig über den zweiten Formabschnitt 2' verteilt.

Die Erfindung sieht jedoch vor, dass die Formnoppen 4d' derart gruppiert sind, dass die Formgruppierung im Wesentlichen kreisförmig um die Längsachse L' verläuft und Gruppierungen parallel zueinander verlaufen. Insbesondere können zumindest 10, vorzugsweise mehr als 20 Formnoppen vorgesehen sein. Diese sind erfindungsgemäß gleichmäßig über den zweiten Formabschnitt 2' verteilt sein.

Das entsprechende Präservativ 3 des Beispiels in Figur 5 würde Noppen 4d aufweisen, die entsprechend ungleichmäßig auf dem zweiten Abschnitt 2 des Präservativs 3 verteilt sind.

Gemäß dem erfindungsgemäßen Herstellungsverfahren kann ein erfindungsgemäßes Präservativ 3 dadurch erhalten werden, dass ein erfindungsgemäßer Formkörper 3' in flüssiges Material, insbesondere flüssiges Latex, eingetaucht wird, wodurch sich ein Materialfilm um den Formkörper 3' ergibt. Zumindest der am tiefsten eingetauchte Bereich des Formkörpers, der dem zweiten Formabschnitt 2' entspricht, weist eine Oberflächenstruktur 4' auf, die der Oberflächenstruktur 4 des Präservativs 3 entspricht. Die Oberflächenstruktur 4 des Präservativs 3 bildet sich aus, indem sich Material um die Oberflächenstruktur 4` des Formkörpers 3` legt und einen Materialfilm ausbildet. Weitere Schritte bei der Herstellung des Präservativs sind das Trocknen, das Vulkanisieren, das Waschen und Trocknen und das Aufrollen des aus dem Materialfilm hergestellten Präservativs unter Ausbilden des Rands.

Die vorliegende Erfindung ist nicht auf die beschriebenen Ausführungsformen beschränkt. Beispielsweise wird der Fachmann erkennen, dass es auch möglich ist, Rippen der Ausführungsform von Fig. 4a und Fig. 4b mit Noppen der Ausführungsform von Fig. 5 zu kombinieren.

Die Merkmale, die in Zusammenhang mit dem Präservativ offenbart sind, treffen entsprechend auf den Formkörper zu. Ebenso ist das Verfahren zur Herstellung durch das erfindungsgemäße Präservativ bzw. den erfindungsgemäßen Formkörper gekennzeichnet.

## Patentansprüche

1. Präservativ (3) für Männer gemäß dem ISO-Standard 4074 (2015) zum Abrollen auf dem Penis, das sich in zumindest einem Zustand im Wesentlichen entlang einer Längsachse (L) erstreckt,
mit einem ersten Abschnitt (1), der in zumindest einem Zustand zylinderförmig ausgebildet ist, und
einem Reservoir als zweiten Abschnitt (2) zur Aufnahme von Sperma,
wobei der zweite Abschnitt (2) an einem geschlossenen Ende des Präservativs (3) entlang der Längsachse (L) betrachtet angeordnet ist, und
ein Übergang (5) vom ersten Abschnitt (1) zum zweiten Abschnitt (2) eine Verjüngung aufweist, sodass sich das Präservativ (3) vom ersten Abschnitt (1) zum zweiten Abschnitt (2) verjüngt,
wobei der zweite Abschnitt (2) zumindest abschnittsweise eine Oberflächenstruktur (4) auf der Außenseite aufweist, bei dem die Oberflächenstruktur (4) mehrere Noppen aufweist und die mehreren Noppen derart gruppiert sind, dass die Gruppierung im Wesentlichen kreisförmig um die Längsachse (L) verläuft und **dadurch gekennzeichnet, dass** die Noppen gleichmäßig und zueinander versetzt angeordnet sind, und die Noppen um weniger als 1 mm vorstehen und weniger als 2 mm breit sind.

2. Präservativ nach Anspruch 1, bei dem die Oberflächenstruktur (4) in einem Bereich des zweiten Abschnitts (2) angeordnet ist, in dem die Oberfläche des zweiten Abschnitts (2) parallel oder im Wesentlichen parallel zur Längsachse (L) verläuft.

3. Präservativ nach einem der vorhergehenden Ansprüche, bei dem zusätzlich mehrere Rippen parallel zueinander verlaufen.

4. Präservativ nach einem der vorhergehenden Ansprüche, bei dem die mehreren Noppen derart in Gruppierungen gruppiert sind, dass mehrere Gruppierungen parallel zueinander verlaufen.

5. Formkörper (3') zum Herstellen eines Präservativs (3) gemäß dem ISO-Standard 4074 (2015) für Männer zum Abrollen auf dem Penis, insbesondere nach einem der vorhergehenden Ansprüche,
wobei sich der Formkörper (3') im Wesentlichen entlang einer Längsachse (L') erstreckt und aufweist:
einen ersten Formabschnitt (1') zum Ausbilden eines ersten Abschnitts (1), der in zumindest einem Zustand zylinderförmig ausgebildet ist, und
einen zweiten Formabschnitt (2') zum Ausbilden eines Reservoirs,
wobei der zweite Formabschnitt (2') an einem geschlossenen Ende des Formkörpers (3') entlang der Längsachse (L') betrachtet angeordnet ist und
der Formkörper einen Formübergang (5') zwischen dem ersten (1') und dem zweiten (2') Formabschnitt aufweist,
wobei der Formübergang (5') eine Formverjüngung vom ersten Formabschnitt (1') zum zweiten Formabschnitt (2') hin aufweist, sodass sich der Formkörper vom ersten Formabschnitt zum zweiten Formabschnitt verjüngt, und der zweite Formabschnitt (2') auf der Außenseite zumindest abschnittsweise eine Oberflächenformstruktur (4') zum Ausbilden einer Oberflächenstruktur (4) auf der Außenseite des Präservativs aufweist, bei der mehrere Formnoppen (4d) derart gruppiert sind, dass eine Formgruppierung im Wesentlichen kreisförmig um die Längsachse (L')verläuft,
**dadurch gekennzeichnet, dass**
die Formnoppen gleichmäßig und zueinander versetzt angeordnet sind und der erste Formabschnitt (1') zum Ausbilden des ersten Abschnitts (1) eingerichtet ist, wobei die Formnoppen um weniger als 1 mm vorstehen und weniger als 2 mm breit sind.

6. Formkörper nach Anspruch 5, bei dem die Oberflächenformstruktur (4') in einem Bereich des zweiten Formabschnitts (2') angeordnet ist, der sich parallel oder im Wesentlichen parallel zur Längsachse (L') erstreckt.

7. Formkörper nach Anspruch 5 oder 6, bei dem zusätzlich mehrere Formrippen parallel zueinander verlaufen.

8. Formkörper nach Anspruch 5, 6 oder 7, bei dem die mehreren Formnoppen (4d) in Formgruppierungen derart gruppiert sind, die mehreren Formgruppierungen parallel zueinander verlaufen.

9. Verfahren zum Herstellen eines Präservativs (3) für Männer, vorzugsweise aus Latex, nach einem der vorhergehenden Ansprüche 1 bis 4, mit den folgenden Schritten:
Bereitstellen eines sich im Wesentlichen entlang einer Längsachse (L') erstreckenden Formkörpers (3') nach einem der vorhergehenden Ansprüche 5 bis 8,
zumindest abschnittsweises Eintauchen des Formkörpers (3') in flüssiges Material, insbesondere Latex, zum Ausbilden eines Material-Films, insbesondere Latex-Films, zumindest abschnittsweise um den Formkörper herum,
Ausbilden einer Oberflächenstruktur (4) im MaterialFilm, insbesondere Latex-Film, zumindest an dem am tiefsten eingetauchten Bereich des Formkörpers.

## Claims

1. Contraceptive (3) for men according to ISO standard 4074 (2015) for rolling on the penis, which in at least one state extends substantially along a longitudinal axis (L),
with a first section (1), which is cylindrically formed in at least one state, and
a reservoir as a second section (2) for receiving sperm,
wherein the second section (2) is arranged at a closed end of the contraceptive (3) as viewed along the longitudinal axis (L), and
a transition (5) from the first section (1) to the second section (2) has a taper, such that the contraceptive (3) tapers from the first section (1) to the second section (2),
wherein the second section (2) has a surface structure (4) on the outside at least in sections, wherein the surface structure (4) has several nubs and the several nubs are grouped in such a way that the grouping runs substantially circularly around the longitudinal axis (L), and **characterised in that** the nubs are arranged uniformly and offset with respect to each other, and the nubs protrude by less than 1 mm and are less than 2 mm wide.

2. Contraceptive according to claim 1, in which the surface structure (4) is arranged in an area of the second section (2) in which the surface of the second section (2) runs parallel or substantially parallel to the longitudinal axis (L).

3. Contraceptive according to any one of the preceding claims, in which additionally several ribs run parallel to each other.

4. Contraceptive according to any one of the preceding claims, in which the several nubs are grouped in groupings such that several groupings run parallel to each other.

5. Mould body (3') for producing a contraceptive (3) according to ISO standard 4074 (2015) for men for rolling on the penis, in particular according to any one of the preceding claims,
wherein the mould body (3') extends substantially along a longitudinal axis (L') and has:
a first mould section (1') for forming a first section (1) which is cylindrically formed in at least one state, and
a second mould section (2') for forming a reservoir,
wherein the second mould section (2') is arranged at a closed end of the mould body (3') as viewed along the longitudinal axis (L') and
the mould body has a mould transition (5') between the first (1') and the second (2') mould section,
wherein the mould transition (5') has a mould taper from the first mould section (1') to the second mould section (2'), such that the mould body tapers from the first mould section to the second mould section, andthe second mould section (2') has a surface mould structure (4') on the outside at least in sections for forming a surface structure (4) on the outside of the contraceptive, in which several mould nubs (4d) are grouped in such a way that a mould grouping runs substantially circularly around the longitudinal axis (L'),
**characterised in that**
the mould nubs are arranged uniformly and offset relative to one another and the first mould section (1') is arranged to form the first section (1), wherein the mould nubs protrude by less than 1 mm and are less than 2 mm wide.

6. Mould body according to claim 5, in which the surface mould structure (4') is arranged in an area of the second mould section (2') which extends parallel or substantially parallel to the longitudinal axis (L').

7. Mould body according to claim 5 or 6, in which additionally several mould ribs run parallel to one another.

8. Mould body according to claim 5, 6 or 7, in which the several mould nubs (4d) are grouped in mould groupings in such a way that the several mould groupings run parallel to each other.

9. Method for producing a contraceptive (3) for men, preferably of latex, according to any one of the preceding claims 1 to 4, having the following steps:
providing a mould body (3') extending substantially along a longitudinal axis (L') according to any one of the preceding claims 5 to 8,
immersing the mould body (3') at least in sections in liquid material, in particular latex, to form a material film, in particular latex film, at least in sections around the mould body,
forming a surface structure (4) in the material film, in particular latex film, at least at the most deeply immersed area of the mould body.

## Revendications

1. Préservatif (3) pour homme selon la norme ISO 4074 (2015) destiné à être déroulé sur le pénis, qui s'étend sensiblement le long d'un axe longitudinal (L) dans au moins un état,
avec un premier segment (1), qui est réalisé de manière cylindrique dans au moins un état, et
un réservoir en tant que deuxième segment (2) destiné à recevoir du sperme,
dans lequel le deuxième segment (2) est disposé, observé le long de l'axe longitudinal (L), sur une extrémité fermée du préservatif (3), et
une transition (5) du premier segment (1) au deuxième segment (2) présente un rétrécissement, de sorte que le préservatif (3) se rétrécit du premier segment (1) au deuxième segment (2),
dans lequel le deuxième segment (2) présente au moins par endroits une structure superficielle (4) sur le côté extérieur, où la structure superficielle (4) présente plusieurs tétons et les plusieurs tétons sont groupés de telle manière que le groupement s'étend sensiblement de manière circulaire autour de l'axe longitudinal (L), et **caractérisé en ce que** les tétons sont agencés de manière homogène et de manière décalée les uns par rapport aux autres, et les tétons font saillie sur moins de 1 mm et présentent une largeur inférieure à 2 mm.

2. Préservatif selon la revendication 1, où la structure superficielle (4) est disposée dans une zone du deuxième segment (2), dans laquelle la surface du deuxième segment (2) s'étend de manière parallèle ou de manière sensiblement parallèle par rapport à l'axe longitudinal (L).

3. Préservatif selon l'une quelconque des revendications précédentes, où en supplément plusieurs nervures s'étendent de manière parallèle les unes par rapport aux autres.

4. Préservatif selon l'une quelconque des revendications précédentes, où les plusieurs tétons sont groupés en groupements de telle manière que plusieurs groupements s'étendent de manière parallèle les uns par rapport aux autres.

5. Corps moulé (3') pour fabriquer un préservatif (3) selon la norme ISO 4074 (2015) pour homme destiné à être déroulé sur le pénis, en particulier selon l'une quelconque des revendications précédentes,
dans lequel le corps moulé (3') s'étend sensiblement le long d'un axe longitudinal (L') et présente :
un premier segment moulé (1') destiné à réaliser un premier segment (1), qui est réalisé de manière cylindrique dans au moins un état, et
un deuxième segment moulé (2') destiné à réaliser un réservoir,
dans lequel le deuxième segment moulé (2') est agencé, observé le long de l'axe longitudinal (L'), sur une extrémité fermée du corps moulé (3'), et
le corps moulé présente une transition moulée (5') entre le premier (1') et le deuxième segment moulé (2'),
dans lequel la transition moulée (5') présente un rétrécissement moulé du premier segment moulé (1') au deuxième segment moulé (2') de sorte que le corps moulé se rétrécit du premier segment moulé au deuxième segment moulé, et le deuxième segment moulé (2') présente sur le côté extérieur au moins par endroits une structure moulée superficielle (4') destinée à réaliser une structure superficielle (4) sur le côté extérieur du préservatif, où plusieurs tétons moulés (4d) sont groupés de telle manière qu'un groupement moulé s'étend de manière sensiblement circulaire autour de l'axe longitudinal (L'),
**caractérisé en ce que**
les tétons moulés sont disposés de manière homogène et de manière décalée les uns par rapport aux autres et le premier segment moulé (1') est mis au point pour réaliser le premier segment (1), dans lequel les tétons moulés font saillie sur moins d'un 1 mm et présentent une largeur inférieure à 2 mm.

6. Corps moulé selon la revendication 5, où la structure moulée superficielle (4') est disposée dans une zone du deuxième segment moulé (2'), qui s'étend de manière parallèle ou de manière sensiblement parallèle par rapport à l'axe longitudinal (L').

7. Corps moulé selon la revendication 5 ou la revendication 6, où en supplément plusieurs nervures moulées s'étendent de manière parallèle les unes par rapport aux autres.

8. Corps moulé selon la revendication 5, la revendication 6 ou la revendication 7, où les plusieurs tétons moulés (4d) sont groupés en des groupements moulés de telle manière que les plusieurs groupements moulés s'étendent de manière parallèle les uns par rapport aux autres.

9. Procédé pour fabriquer un préservatif (3) pour homme, de préférence à partir de latex, selon l'une quelconque des revendications précédentes 1 à 4, avec les étapes suivantes :
la fourniture d'un corps moulé (3') s'étendant sensiblement le long d'un axe longitudinal (L') selon l'une quelconque des revendications précédentes 5 à 8,
l'immersion au moins par endroits du corps moulé (3') dans du matériau liquide, en particulier du latex, pour réaliser un film de matériau, en particulier un film de latex, tout autour au moins par endroits du corps moulé,
la réalisation d'une structure superficielle (4) dans le film de matériau, en particulier le film de latex, au moins sur la zone immergée la plus profondément du corps moulé.
